(19) Europäisches Patentamt
European Patent Office
Office européen des brevets

(11) Veröffentlichungsnummer: **0 002 001**
B1

(12) # EUROPÄISCHE PATENTSCHRIFT

(45) Veröffentlichungstag der Patentschrift: **01.04.81**

(51) Int. Cl.³: **C 07 D 233/78,** C 07 D 233/86, C 08 G 18/78, C 08 G 18/80

(21) Anmeldenummer: **78101269.5**

(22) Anmeldetag: **02.11.78**

(54) **Heterocyclische Polyisocyanate, ihre Herstellung und Verwendung.**

(30) Priorität: **12.11.77 DE 2750722**

(43) Veröffentlichungstag der Anmeldung: **30.05.79 Patentblatt 79/11**

(45) Bekanntmachung des Hinweises auf die Patenterteilung: **01.04.81 Patentblatt 81/13**

(84) Benannte Vertragsstaaten: **DE FR GB**

(56) Entgegenhaltungen: **DE-A-1 670 812**

(73) Patentinhaber: **BAYER AG, Zentralbereich Patente, Marken und Lizenzen, D-5090 Leverkusen 1, Bayerwerk (DE)**

(72) Erfinder: **Lewalter, Jürgen, Dr., Bergstrasse 88, D-5068 Odenthal (DE)**
Erfinder: **Merten, Rudolf, Dr., Berta-von-Suttner-Strasse 55, D-5090 Leverkusen 1 (DE)**
Erfinder: **Dünwald, Willi, Dr., Geschwister-Scholl-Strasse 16, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

EP 0 002 001 B1

ACTORUM AG.

# Heterocyclische Polyisocyanate, ihre Herstellung und Verwendung

Die Erfindung betrifft Polyisocyanate mit mindestens einem durch Carbonsäureestergruppen substituierten Hydantoin- oder Thiohydantoinring im Molekül, deren Herstellung durch Umsetzung von Äthylen-1,2-dicarbonsäureestern, Aminoalkoholen und einem Überschuss an Polyiso(thio)cyanaten, sowie deren Verwendung zur Herstellung hitzebeständiger Beschichtungsmittel.

Verfahren zur Herstellung von Hydantoinen (J. Am. Chem. 45/383) bzw. Polyhydantoinen (BE 678 282) sind bekannt. Auch die Hydantoinherstellung durch Umsatz von Äthylen-1,2-dicarbonsäureestern, Aminen und Isocyanaten ist beschrieben worden. Dabei werden aber in der Regel äquivalente Mengen der Reaktionspartner eingesetzt, da man bei Verwendung eines Überschusses eines Reaktionspartners, insbesondere des Polyisocyanats befürchten musste, dass die Polyisocyanate in Gegenwart der Amine bevorzugt zu Polyisocyanuraten und nicht unter Ringschluss zu den gewünschten Hydantoinen reagieren.

Überraschenderweise wurde nun gefunden, dass trotz eines Überschusses an Polyisocyanaten die Herstellung von mit Carbonsäureestergruppen substituierten Hydantoinen reibungslos verläuft und man dabei neue hydantoingruppenhaltige Polyisocyanate erhält, die wertvolle Zwischenprodukte darstellen.

Gegenstand der Erfindung sind daher neue mit Estergruppen modifizierte hydantoingruppenhaltige Polyiso(thio)cyanate der allgemeinen Formel (I)

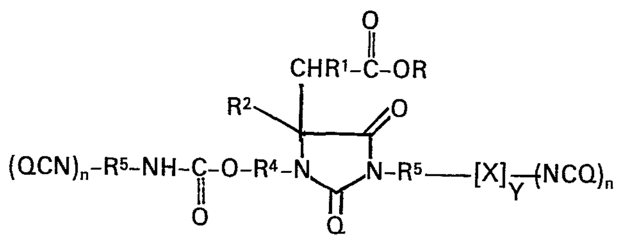

(I)

in der X für die wiederkehrenden Einheiten (II) und/oder (III)

(II) 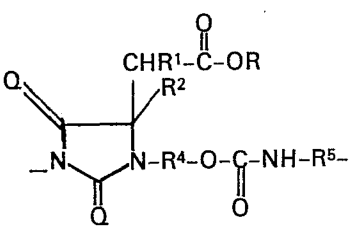

(III) 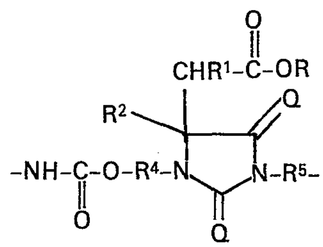

und

R für einen aliphatischen Rest mit $C_1$-$C_{20}$, der über Äther-, Ester-, Urethan-, Harnstoff-, Amid-, Imid-, Amidimid-, Esterimid-, Esteramidimid-, Epoxidgruppen in Polymere eingebaut sein kann oder mit $R^1$ bzw. $R^2$ einen Ring mit bis zu 8 Ringgliedern bildet, steht,

$R_1$ und $R_2$ gleich oder verschieden, Wasserstoff, Halogen, einen Alkylrest mit $C_1$-$C_6$ oder Cycloalkylrest mit $C_5$-$C_8$, Alkylarylrest mit $C_7$-$C_{16}$, einen Arylrest mit $C_6$-$C_{20}$ bedeuten oder zusammen einen Ring mit bis zu 8 Ringgliedern bilden,

$R_4$ für einen aliphatischen Rest mit $C_2$-$C_{10}$, einen cycloaliphatischen Rest mit $C_4$-$C_{16}$, einen araliphatischen Rest mit $C_7$-$C_{16}$, einen aromatischen Rest mit $C_6$-$C_{20}$, oder einen ein oder mehrere Heteroatome wie N, O oder S enthaltenden Aryl- oder Cycloalkylrest mit $C_5$-$C_{12}$, die alle mit Halogen- und/oder Alkylgruppen mit $C_1$-$C_6$ substituiert sein können, steht,

$R_5$ für einen aliphatischen Rest mit 2-20 C-Atomen, einen aromatischen Rest mit 5-12 C-Atomen, einen cycloaliphatischen Rest mit 5-12 C-Atomen, einen aliphatischaromatischen Rest mit 6-20 C-Atomen oder einen ein oder mehrere Heteroatome wie N, O oder S enthaltenden aromatischen oder cycloaliphatischen Rest mit 5-12 C-Atomen steht, die alle durch Halogen, Alkyl- mit $C_1$-$C_6$ und/oder Arylgruppen mit $C_6$-$C_{16}$ substituiert sein können,

Y 0 oder eine ganze Zahl von 1-20,
Q Sauerstoff oder Schwefel und
n eine ganze Zahl von 1-3 bedeuten.

Ein weiterer Gegenstand der Erfindung ist ein Verfahren zur Herstellung von Polyiso(thio)cyanaten, die mit Carbonsäureestergruppen substituierte Hydantoinringe enthalten, durch Umsetzung von gegebenenfalls substituierten, gegebenenfalls in Polymeren eingebauten Äthylen-1,2-dicarbonsäureestern mit primären Aminoalkoholen zu den entsprechenden Asparaginsäureestern und gegebenenfalls gleichzeitige und/oder anschliessende Reaktion mit Polyisocyanaten, das dadurch gekennzeichnet ist, dass pro Mol Asparaginsäureester mindestens 2,1 Äquivalente Isocyanat eingesetzt werden.

Der Reaktionsverlauf kann schematisch durch folgende Formelgleichung wiedergegeben werden:

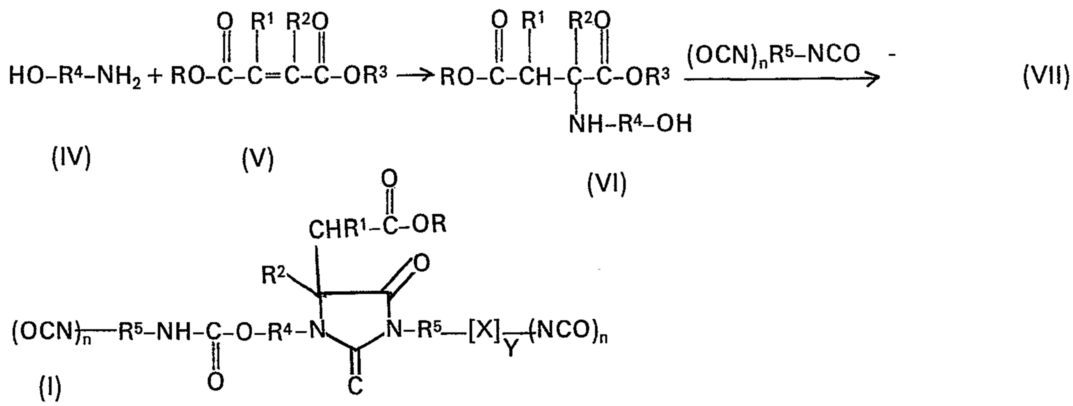

(IV) (V) (VI) (VII) (I)

In der Formelgleichung haben
R, $R^1$, $R^2$, $R^4$, $R^5$, X und Y die oben angegebene Bedeutung,
n steht für eine ganze Zahl von 1-3 und
$R^3$ hat die Bedeutung von R, muss aber nicht identisch mit R sein.

Zur Herstellung der erfindungsgemässen Polyiso(thio)cyanate werden, gegebenenfalls über die Estergruppen polymerisierte, OH-gruppenhaltige Asparaginsäureester eingesetzt, die in situ oder in einer vorgeschalteten Reaktion aus, gegebenenfalls in Polymeren eingebauten 1,2-Dicarbonsäureestern und primären Aminoalkoholen in bekannter Weise zugänglich sind. Die ungesättigten Dicarbonsäureester erhält man durch Umsetzung der freien Säuren oder aus dem Anhydrid durch Veresterung mit Monoalkoholen wie Methanol, Äthanol, Butanol oder Cyclohexanol, Benzylalkohol, Äthylen-, Diäthylenglykolmonoäther, Phenol oder auch Polyalkoholen wie Äthylenglykol und/oder Glycerin, gegebenenfalls in Abmischung mit weiteren Polycarbonsäuren, gegebenenfalls im Gemisch mit Monoalkoholen oder mit hydroxyhaltigen Polyestern,
R und $R^3$ in den oben angegebenen Formeln V und allen folgenden Formeln ist daher ein aliphatischer Rest mit besonders bevorzugt $C_1$–$C_{10}$, der mit OH-Gruppen substituiert bzw. über Äther-, Ester-, Urethan-, Harnstoff-, Amid-, Imid-, Amidimid-, Esterimid-, Esteramidimid-, Epoxidgruppen usw. in Polymere eingebaut sein kann, oder mit $R^1$ bzw. $R^2$ einen Ring mit bis zu 8 Ringgliedern bildet.

Neben den Malein- bzw. Fumarsäureestern können auch vorzugsweise Halogen- oder $C_1$–$C_6$ alkylsubstituierte Äthylen-1,2-dicarbonsäureester, vorzugsweise Methyl-, Dimethyl-, Äthyl-, Butyl-, Phenyl oder $\Delta^1$-Cyclohexan-1,2-dicarbonsäureester in jeder isomeren Form zur Reaktion mit den Aminoalkoholen gebracht werden. Gegebenenfalls kann eine cis/trans-Isomerisierung durch Wärme, Basen und/oder Halogene erzielt werden.

Besonders bevorzugt verwendet werden Ester der Malein- oder Fumarsäure mit Methanol, Äthanol, Butanolen, Benzylalkoholen, Phenolen, Glykoläther, sowie die Monoester mit Äthylen- und Diäthylenglykol, Glycerin.

$R_4$ bedeutet in der allgemeinen Formel IV der Aminohydroxyverbindung einen aliphatischen Rest mit $C_2$–$C_{10}$, einen cycloaliphatischen Rest mit $C_4$–$C_{16}$, einen aromatischen Rest mit $C_7$–$C_{16}$ einen aromatischen Rest mit $C_6$–$C_{20}$ oder einen ein oder mehrere Heteroatome wie N, O oder S enthaltenden Aryl- oder Cycloalkylrest mit $C_5$–$C_{12}$, die alle mit Halogen und/oder Alkylgruppen mit $C_1$–$C_6$ substituiert sein können. Besonders bevorzugt steht $R^4$ für einen Alkylrest mit $C_2$–$C_6$, einen Cycloalkylrest mit $C_4$–$C_{10}$ oder einen Arylrest mit $C_6$–$C_{10}$. Als besonders bevorzugt verwendete Vertreter seien Äthanolamin, Propanolamin, Butanolamin, Aminomethylphenyl oder Aminophenol genannt.

Die Addition der Hydroxyaminoverbindungen an die Äthylen-1,2-dicarbonsäureester erfolgt im allgemeinen bei Temperaturen von -30°C bis 100°C, bevorzugt bei -10°C bis 70°C, um eine Aminolyse der in dem jeweiligen Molekül noch vorhandenen Estergruppierungen zum Amid, gegebenenfalls auch Imid, zu vermeiden.

Gegebenenfalls können bei der Addition Katalysatoren wie Kaliumcarbonat, Trialkylamin, Endoäthylenpiperazin, Essigsäure u.a. mitverwendet werden. Bei weniger löslichen bzw. höher schmelzenden Komponenten wird die 1. Reaktionsstufe im allgemeinen zweckmässigerweise in organischen Lösungsmitteln durchgeführt, wie sie auch für die Folgereaktionen verwendet werden können und anschliessend beschrieben sind. Die Mengenverhältnisse zwischen α,β-ungesättigten Carbonsäureestern und Aminoalkoholen sollten zweckmässig stöchiometrisch gewählt werden. Überschüssige Anteile der einen oder anderen Komponente können jedoch eingesetzt und durch an sich bekannte, erfindungsgemäss nicht erfasste Folgereaktionen in die Polymeren eingebaut werden.

Anstelle der in situ hergestellten hydroxylgruppen-haltige Asparaginsäureester können natürlich auch auf beliebigen Wegen hergestellte, gegebenenfalls durch Kristallisation und/oder Destillation gereinigte Vertreter dieser Substanzklasse eingesetzt werden.

Als erfindungsgemäss einzusetzende Polyisocyanate kommen aliphatische, cycloaliphatische, araliphatische, aromatische und heterocyclische Polyisocyanate in Betracht (vgl. Annalen 562, Seiten 75 bis 136), beispielsweise Äthylen-diisocyanat, 1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanato-methyl-cyclohexan (DAS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-2,2',4"-triisocyanat, Polyphenylpolymethylen-polyisocyanate, wie sie durch Anilin-Formaldehyd-Kondensation und anschliessende Phosgenierung erhalten und z.B. in den britischen Patentschriften 874 430 und 848 671 beschrieben werden, perchlorierte Arylpolyisocyanate, wie sie z.B. in der deutschen Auslegeschrift 1 157 601 beschrieben werden, Carbodiimidgruppen aufweisende Polyisocyanate, wie sie in der deutschen Patentschrift 1 092 007 beschrieben werden, Diisocyanate, wie sie in der US-Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in den deutschen Patentschriften 1 022 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der US-Patentschrift 3 394 164 beschrieben werden, acylierte Harnstoffgruppen aufweisende Polyisocyanate gemäss der deutschen Patentschrift 1 230 778, Biuretgruppen aufweisende Polyisocyanate, wie sie z.B. in der deutschen Patentschrift 1 101 394, in der britischen Patentschrift 889 050 und in der französischen Patentschrift 7 015 514 beschrieben werden, durch Telomerisationsreaktionen hergestellte Polyisocyanate, wie sie z.B. in der beglischen Patentschrift 723 640 beschrieben werden, Estergruppen aufweisende Polyisocyanate, wie sie z.B. in den britischen Patentschriften 956 474 und 1 072 956, in der US-Patentschrift 3 567 763 und in der deutschen Patentschrift 1 231 688 genannt werden, Umsetzungsprodukte der obengenannten Isocyanate mit Acetalen gemäss der deutschen Patentschrift 1 072 358.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Vorzugsweise eignen sich Polyisocyanate der allgemeinen Formel

$$R^5(NCQ)_{n+1} \qquad (VII)$$

in denen $R^5$, definiert wie in der allgemeinen Formel (I), vorzugsweise einen, gegebenenfalls substituierten aliphatischen Rest mit 2–20 C-Atomen, einen aromatischen Rest mit 5–12 C-Atomen, einen cycloaliphatischen Rest mit 5–12 C-Atomen, einen aliphatisch-aromatischen Rest mit 6–20 C-Atomen und einen ein oder mehrere Heteroatome wie N, O oder S enthaltenden aromatischen oder cycloaliphatischen Rest mit 5–12 C-Atomen steht. n ist eine ganze Zahl von 1–3, vorzugsweise 1–2, besonders bevorzugt 1 und Q ist analog oben definiert.

Die Reste können alle durch Halogen, Alkyl- mit $C_1$–$C_6$ und/oder Arylgruppen mit $C_6$–$C_{16}$ substituiert sein.

Bevorzugt verwendet werden die technisch leicht zugänglichen Gemische aus Toluylendiisocyanaten, m-Phenylen-diisocyanat, sowie phosgenierte Kondensate aus Anilin und Formaldehyd mit Polyphenylen-methylen-struktur und die symmetrischen Verbindungen 4,4'-Diisocyanatodiphenylmethan, 4,4'-Diisocyanatodiphenyläther, p-Phenylendiisocyanat und 4,4'-Diisocyanatodiphenyldimethylmethan, Isophorondiisocyanat sowie 4,4'-Diisocyanatodiphenylsulfid und Hexamethylendiisocyanat.

Die Isocyanate können in freier Form, ferner zum Teil oder vollständig auch in Form ihrer beim Umsatz mit reaktiven Wasserstoff enthaltenden Verbindungen zugänglichen und unter den Reaktionsbedingungen als Isocyanat-Abspalter reagierenden Derivate eingesetzt werden.

Vorzugsweise werden als Abspalter die aus aromatischen und aliphatischen Mono- und Polyhydroxy-Verbindungen enthaltenden Carbamidsäureester und die Additionsprodukte aus Lactamen, Oximen und CH-aciden Verbindungen eingesetzt.

Als Beispiele seien die Carbamidsäureester aus Phenol, isomeren Kresolen, deren technischen Gemischen und ähnlichen aromatischen Hydroxylverbindungen, aliphatische Monoalkohole wie Methanol, Äthanol, Propanol, Isopropanol, Butanol, Isobutanol, Cyclohexanol, Allylalkohol, Benzylalkohol und aliphatische Di- oder Polyole wie Äthylenglykol und Trimethylolpropan, weiterhin die Additionsprodukte mit Pyrrolidon-(2), Caprolactam, Butanon-oxim, Malonester, Acetessigester und Acetophenon aufgeführt. Ferner kommen hier die Monoäther von Äthylen- und Diäthylenglykol bzw. Propylen- und Dipropylenglykol mit Methanol, Äthanol, Butanol, Benzylalkohol oder Phenolen in Frage.

Die Isocyanat-Abspalter können als solche eingesetzt oder erst in situ durch Umsetzung mit den entsprechenden Reaktanten erzeugt werden.

Anstelle der genannten (Poly)Isocyanate können auch die analogen (Poly)Isothiocyanate als Ausgangsmaterialien benutzt werden.

Die Mengenverhältnisse zwischen den OHgruppenhaltigen Asparaginsäureester und Polyisocyanaten müssen so gewählt werden, dass die erhaltenen Reaktionsprodukte überschüssige Isocyanatgruppen, möglichst über 10%, vorzugsweise über 30%, gegebenenfalls in verkappter Form, enthalten sollen. Hierzu ist es erforderlich,

dass pro Mol an eingesetztem Asparaginester mit zwei im Sinne der Zerewitinoff-Reaktion reaktiven H-Atome mehr als 2 Äquivalente an Isocyanatgruppen und maximal 2 Mol der einzusetzenden Polyisocyanatkomponente verwendet werden müssen, wobei allerdings überschüssiges eingesetztes Polyisocyanat, welches gegebenenfalls anschliessend entfernt oder auch als zusätzliches nicht erfindungsgemässes Polyisocyanat im Reaktionsprodukt belassen werden kann, nicht stört. Es sollten mindestens 2,1, vorzugsweise mindestens 2,2 Äquivalente Isocyanat pro Mol N-Hydroxyalkylasparaginsäureestereinheit eingesetzt werden.

Die erfindungsgemässe Umsetzung der hydroxygruppen-haltigen Asparaginsäureester mit den Polyisocyanaten zu den beanspruchten Polyhydantoingruppen enthaltenden Polyisocyanaten erfolgt gegebenenfalls in Lösungsmitteln, die gegenüber den Reaktionskomponenten inert sind oder aber speziell gegenüber der Polyisocyanatkomponente zu Verbindungen mit spaltbaren Additionsverbindungen befähigt sind, z.B. Phenole, Lactame, Alkohole, Kohlenwasserstoffe, Halogenkohlenwasserstoffe, Ester, cyclische Ester, Ketone, Äther, substituierte Amide, Nitrile. Genannt seien beispielsweise Phenol, techn. Kresolgemische, ε-Caprolactam, Acetophenon, Äthylenglykolbutyläther, Diäthylglykolmethyläther, Cyclohexanon, Glykolmonomethylätheracetat, γ-Butyrolacton, ε-Caprolacton, Benzoesäurealkylester, N-Methylpyrrolidon, Dimethylformamid, Dimethylacetamid, Benzonitril und andere sowie deren Gemische.

Zur Umsetzung der Asparaginsäureester mit den Polyisocyanaten werden die Reaktionskomponenten, gegebenenfalls mit den Lösungsmitteln einige Minuten bis zu mehreren Stunden bei Temperaturen von −30 °C bis 350 °C, bevorzugt −10 °C bis 250 °C, gehalten. Der Reaktionsverlauf kann über die IR-Spektren verfolgt werden.

Die hohe Geschwindigkeit der erfindungsgemässen Umsetzung gestattet auch deren Durchführung in Gegenwart phenolischer und/oder alkoholischer Hydroxylgruppen und/oder Amidgruppen, d.h. derartige Gruppierungen können in den Umsetzungskomponenten, gegebenenfalls als Blockmittel, durchaus vorhanden sein, bzw. überschüssige Hydroxyl- bzw. Lactam-Komponenten können auch als Lösungsmittel verwendet werden.

Die Durchführung dieser Reaktion kann gegebenenfalls unter Inertgas vorgenommen werden.

Die Acidität saurer Lösungsmittel wie der Phenole bzw. Kresole genügt zur Reaktionsführung innerhalb hinreichend kurzer Reaktionszeiten. In inerten Medien oder in Schmelzansätzen können beispielsweise Carbonsäuren mit hinreichendem Schmelz- bzw. Siedepunkt wie Essigsäure, Benzoesäure, Bernsteinsäure, Benzoldicarbonsäuren, Butantetracarbonsäure, Trimellitsäure bzw. deren Anhydride gegebenenfalls auch als einbaufähige Katalysatoren in Mengen von 0,1 bis 40, vorzugsweise von 1 bis 10 Prozent in Val, bezogen auf ein Val Isocyanat, mitverwendet werden.

Zur weiteren Beschleunigung der ablaufenden Reaktionen sind die aus der Isocyanatchemie geeigneten bekannten Basen, Säuren und/oder Metallkatalysatoren wie Triäthylamin, N-Methylmorpholin, Endoäthylenpiperazin, Titantetrabutylat, Titanaminoalkoholat, Eisenacetylacetonat, Dibutylzinndilaurat, Bleioctoat, Essigsäure oder p-Toluolsulfonsäure zu verwenden.

Wie bereits erwähnt, kann die erfindungsgemässe Umsetzung auch in situ aus den einzelnen Komponenten, d.h. den Aminoalkoholen, den α,β-ungesättigten 1,2-Dicarbonestern und den gegebenenfalls latent blockierten Polyisocyanaten in einem Reaktionsschritt durchgeführt werden. Eine weitere Variante des Verfahrens besteht in einer dergestalt verlaufenden Reaktionsfolge, dass die aus der Aminoalkoholkomponente und den gegebenenfalls latent blokkierten Polyisocyanaten erhaltenen primären Additionsprodukte durch Zugabe der gegebenenfalls in Polymeren eingebauten α,β-ungesättigten 1,2-Dicarbonester in die erfindungsgemässen Hydantoingruppen enthaltenden gegebenenfalls blockierten Polyisocyanate übergeführt werden (indem sich unter den Reaktionsbedingungen der erfindungsgemässen Umsetzung offensichtlich Gleichgewichte der entsprechenden Reaktionskomponenten einstellen). Ferner können auch die in Polymere, z.B. Polyester, Polyurethan, Polyamiden, Polyimiden, Polyesterimiden, Polyamidimiden, Polyesteramidimiden, Polyepoxiden usw. eingebauten α,β-ungesättigten -1,2-Dicarbonsäureester mit Aminoalkoholen und Polyisocyanaten zu den erfindungsgemässen Hydantoingruppen enthaltenden Verbindungen umgesetzt und eingebaut werden.

Die erfindungsgemässen Hydantoingruppen enthaltenden Polyisocyanate stellen hochviskose bis spröde Massen dar, die je nach ihrem Anwendungszweck in konzentrierter Form oder auch als Lösungen in den bereits früher aufgeführten Lösungsmitteln vorliegen können.

Die erfindungsgemässen Hydantoingruppen enthaltenden Polyisocyanate werden zur Herstellung von Polyurethankunststoffen, vorzugsweise von Elektroisolierstoffen, eingesetzt. Hierbei können die erfindungsgemässen Polyisocyanate direkt mit den üblichen Polyestern, Polyesterimiden, Polyamidimiden, Polyesteramidimiden, Polyesterhydantoinen unter Ausbildung von Carbonester- und Carbamidesterstrukturen nach den bekannten Methoden eingebrannt werden. Eine weitere Variante für die beschriebenen Polyisocyanate besteht darin, dass sie als Polyester- und Polyisocyanatkomponente in den üblichen Verfahren zur Herstellung von gegebenenfalls Estergruppen aufweisenden Polyimiden, Polyamidimiden und Polyhydantoinen eingesetzt werden können. Diese Reaktion kann sowohl in einer vorgeschalteten Operation als auch in situ zusammen mit dem Einbrennvorgang vorgenommen werden. Die hierzu angewendeten Verfahrensweisen gehen beispielsweise aus den BE 678 282, DP 1 035 362 hervor.

Beispiel 1:

a)

732 g Carbitol (Diäthylenglykolmonoäthyläther)

25 g ε-Caprolactam im Vakuum bei ≦70 °C entgasen, unter $N_2$ bei 30 °C in einem Schub

750 g 4,4'-Diisocyanatodiphenylmethan, dann bei 100 °C in einem Schub

552,5 g 4,4'-Diisocyanatodiphenylmethan, 1 h 170 °C nachrühren, dann bei 30–45 °C

571 g N-ω-Hydroxypropylasparaginsäuredimethylester zutropfen, ½ h bei 45 °C nachrühren und schliesslich bei 175–200 °C ca. 83 g Methanol austragen.

Das tiefbraune Harz besitzt im IR die typischen Hydantoinbanden, hat einen latenten NCO-Gehalt von 9,0 Gew.-% und besitzt in 70 gew.-%iger Lösung in Carbitol eine Viskosität von ca. 850 $cP_{20°C}$.

b)

1000 g der 70 gew.-%igen Harzlösung 1a werden mit

350 g eines Polyesters aus 1,8 Mol Phthalsäure, 1,8 Mol Trimethylolpropan und 1,4 Mol Äthylenglykol mit 12 Gew.-% OH-Gruppen,

10 g Soligen Zink (ca. 8 Gew.-% Zn) mit gleichen Teilen Benzylalkohol, Äthylencarbonat, Diacetonalkohol und Xylol zu einer insgesamt 50 Gew.-%igen Lösung verarbeitet, deren Viskosität 29 Sekunden im DIN-4-Becher beträgt.

Der mit dieser Lacklösung auf einem 0,7 mm Cu-Draht in einem 3,5 m Ofen bei 380 °C applizierte Lackfilm besitzt eine Erweichungstemperatur von ca. 250 °C bei ausreichender Elastizität und einer Hitzeschockfestigkeit von 180 °C.

Beispiel 2:

1463 g Carbitol,

41 g ε-Caprolactam, werden i.V. bei ≦70 °C entgast, dann unter $N_2$ bei 30 °C in einem Schub zunächst mit

750 g 4,4'-Diisocyanatodiphenylmethan versetzt, homogenisiert, dann bei 100 °C in einem Schub mit

552,5 g 4,4'-Diisocyanatodiphenylmethan verrührt und 1 h bei 170 °C bis zum völligen Umsatz des Isocyanates gehalten.

Darauf rührt man bei 100 °C entweder

1142 g N-ω-Hydroxypropyl-asparaginsäuredimethylester oder nacheinander

390,5 g ω-Aminopropanol und

751,0 g Maleinsäuredimethylester ein, homogenisiert 1 h bei 130 °C und fügt anschliessend

500,2 g Trimellitsäureanhydrid hinzu, trägt bei 175–200 °C ca. 167 g Methanol aus und kondensiert bei 200–230 °C bis zum Ende der $CO_2$-Entwicklung.

Die heisse Harzschmelze wird mit

920 g Carbitol auf 50% verdünnt und besitzt nach Zusatz von

23 g Titantetrabutylat in

46 g Acetylaceton eine Viskosität von ca. 5800 $cP_{20°C}$, ist aber nach Verdünnung mit Xylol oder Solvesso auf einen ca. 45%igen Festgehalt direkt fahrbar.

Ein aus dieser Lacklösung beschichteter und in einem 4 m Ofen bei 10 m/Minute eingebrannter 0,7 mm Cu-Draht besitzt eine Erweichungstemperatur von >330 °C, einen Hitzeschock von >220 °C und übersteht ca. 7 Tage 200 °C.

Beispiel 3:

a)

1813 g Toluylendiisocyanat (Isomerengemisch aus 2,4:2,6 wie 80:20) werden bei Raumtemperatur unter $N_2$ mit einem Gemisch aus

1143 g Benzylalkohol und

389 g N-ω-Hydroxypropylasparaginsäuredimethylester vermischt, dann bei 70–80 °C bis zu einem Rest-NCO-Gehalt von ≦8,4 Gew.-% gerührt und schliesslich ab 100 °C mit

300,5 g Trimethylolpropan verrührt.

Abschliessend rührt man bei 150–175 °C bis zum völligen Isocyanatverbrauch und trägt dabei ca. 50 g Methanol aus.

Die resultierende hellbraune Schmelze besitzt einen Fp: 66 °C, eine Viskosität von ca. 998 mPa·$s_{150°C}$, einen latenten NCO-Gehalt von ca. 12,0 Gew.-%.

b)

1200 g der unter a) hergestellten Schmelze werden mit

600 g des in Beispiel 1b) angeführten Polyesters

60 g ε-Caprolactam und

20 g Vulkazit 576® (Bayer AG) bei 150 °C unter $N_2$ homogenisiert, anschliessend aus einem 150–160 °C heissen Lackierbad auf einen ca. 0,7 mm Cu-Draht appliziert und in einem 4 m Ofen bei ca. 400 °C eingebrannt.

Der bei einer Abzugsgeschwindigkeit von 10 m lackisolierte Cu-Draht besitzt eine Erweichungstemperatur von 240–250 °C, einen Hitzeschock von ca. 180 °C bei hoher Elastizität, guter Schabefestigkeit und einer Lötzeit von ca. 2 Sekunden bei 370 °C. Das Schmelzharz selbst hat einen Fp von 50 °C und eine Viskosität von 358 mPa·$s_{150°C}$.

Beispiel 4:

a)

403 g Diäthylenglykol,

219,2 g N-ω-Hydroxypropylasparaginsäuredimethylester und

536 g Trimethylolpropan werden homogenisiert und ab 25 °C direkt mit

700 g Toluylendiisocyanat (Isomerengemisch aus 2,4:2,6 wie 80:20), dann ab 100 °C nochmals mit

483,2 g Toluylendiisocyanat vermischt. Anschliessend rührt man noch ca. 2 h bei 175 °C und trägt dabei 30 g Methanol aus.
Das bei Raumtemperatur spröde, honigfarbene Harz besitzt einen Fp: 92–95 °C, eine Viskosität von 1610 $mPa \cdot s_{20°C}$, 50%ig in Methylglykolacetat, sowie einen Hydroxylgehalt von ca. 5,8 Gew.-%.

b)

500 g eines aus 3 Mol Toluylendiisocyanat, 3 Mol Phenol und 1 Mol Trimethylolpropan erhaltenen Isocyanatabspalters

330 g Harz 4a)

170 g eines Polyesters aus 2,2 Mol Terphthalsäure, 3,9 Mol Äthylenglykol und 1,0 Mol Glycerin mit ca. 6,0 Gew.-% OH-Gruppen

10 g Octa-Soligen Zink (8% Zn) werden zu 30 Gew.-% in einem Lösungsmittelgemisch aus gleichen Teilen Benzylalkohol, Äthylglykolacetat, Carbitol und Xylol zu einer Lösung von ca. 27 Sek. Durchlaufzeit im DIN-4-Becher verarbeitet.
Mit dieser Lacklösung kann mit einer Aumann-Drahtlackiermaschine FLK 240® bei einer Ofentemperatur von 450/500 °C ein 0,1 mm Cu-Draht mit einer Abzugsgeschwindigkeit von 450/400 m lackiert werden. Die Lötzeit beträgt ca. 1 Sekunde bei 360 °C, der tg δ liegt bei >150 °C.

Beispiel 5:

144 g Maleinsäuredimethylester werden unter $N_2$ bei −10 bis ≦0 °C mit

75 g ω-Aminopropanol verrührt, nach 2 Std. bei 25 °C mit

500 g γ-Butyrolacton vermischt und schliesslich ab 20 bis ≦30 °C mit der Lösung von

500,4 g 4,4′-Diisocyanatodiphenylmethan in

200 g Toluol kondensiert. Man rührt 1 Stunde bei 25 °C bzw. bis zu einem NCO-Gehalt von ≦7,5 Gew.-%, vermischt dann mit

186 g Äthylenglykol und trägt in ca. 3 Stunden bei langsam bis schliesslich auf 20 °C gesteigerten Temperaturen mindestens 32 g Destillat aus. Danach werden bei 120 °C insgesamt

576,4 g Trimelitsäureanhydrid zugesetzt und in 8 Std. bei Temperaturen bis zu 220 °C insgesamt noch mindestens 86 g Destillat ausgetragen. Dabei wird bei zunehmender Viskosität mit schliesslich insgesamt

1600 g γ-Butyrolacton auf eine Endviskosität von ca. 1450 $mPa \cdot s_{20°C}$ verdünnt und abschliessend bei 100 °C mit

12,5 g Titantetrabutylat, gelöst in Acetylaceton, vermischt.

Das nach einer Methanolfällung verbleibende Bindemittel besitzt die den Hydantoinen und Amidimiden eigenen IR-Banden.
Der mit der Lacklösung in einem 4 m Ofen bei 9 m/Minute auf einen 0,7 mm Cu-Draht aufgebrachte Lackfilm besitzt eine Erweichungstemperatur von >330 °C, einen Hitzeschock von >260 °C und eine gute Lösungsmittelfestigkeit.

Beispiel 6:

a)

357,7 g Maleinsäureanhydrid,

540,2 g Phthalsäureanhydrid,

671,6 g Glycerin und

334,8 g Glykol werden vermischt, in 36 Std. von RT auf ca. 20 °C geheizt und dabei insgesamt 156 g $H_2O$ ausgetragen.

Das hellgelbe Harz besitzt ca. 13,4% OH-Gruppen, eine Säurezahl von 1,5 und eine Hydrierzahl von 0,64.

b)

633 g des Harzes 6a) werden mit

633 g Äthyldiglykol homogenisiert und unter $N_2$ ab 20 bis ≦25 °C mit

75 g ω-Propanolamin vermischt und bei 50 °C bis zum Verbrauch der C = C-Doppelbindungen gerührt.
Zu dieser Harzlösung gibt man eine Lösung von

1270 g eines durch Kondensation von 3 Mol Toluylendiisocyanat mit 1 Mol Trimethylolpropan und nachträglichem Verschliessen der freien Isocyanatgruppen durch 3 Mol Phenol erhaltenen Isocyanatabspalter in

1200 g Äthyldiglykol und

1134 g Xylol.

Die resultierende Lacklösung besitzt eine Viskosität von ca. 27 Sekunden im Ford-4-Becher.
Die Lackierung eines 0,7 mm Cu-Drahtes mit obiger Lacklösung in einem 3 m-Ofen von ca. 320 °C Ofentemperatur liefert bei einer Abzugsgeschwindigkeit von 10 m/Minute einen elastischen Lackfilm von guter Oberflächenhärte mit einer Erweichstemperatur von ca. 260 °C, einem Hitzeschock von gut 180 °C sowie einem

tgδ-Knickpunkt von ca. 150 °C und einer Lötzeit von <1 Sekunde bei 370 °C.

Beispiel 7:

| | | |
|---|---|---|
| 348,3 | g | Toluylendiisocyanat (Isomerengemisch aus 2,4:2,6 wie 80:20) in |
| 200 | g | Toluol werden unter $N_2$ ab 20 bis ≦40 °C mit |
| 219,2 | g | N-ω-Hydroxypropylasparaginsäuredimethylester kombiniert und 1 Std. bei 40 °C homogenisiert. Anschliessend kondensiert man bei 120 bis schliesslich 175 °C unter Destillation von ca. 32 g Methanol. |

Das resultierende spröde, honiggelbe Harz besitzt eine Viskosität von ca. 87 000 mPa·$s_{160°C}$ und einen NCO-Gehalt von 15,2 Gew.-%.

Beispiel 8:

a)

| | | |
|---|---|---|
| 348,3 | g | Toluylendiisocyanat (Isomerengemisch aus 2,4:2,6 wie 80:20) in |
| 200 | g | Xylol werden unter $N_2$ ab 20 bis ≦40 °C mit |
| 219,2 | g | N-Hydroxypropylasparaginsäuredimethylester vermischt und ca. 1 Stunde bei 40 °C homogenisiert. Anschliessend trägt man zunächst bei Temperaturen bis zu 180 °C 32 g Methanol aus und versetzt dann bei einem NCO-Gehalt von <15 Gew.-% ab 150–120 °C mit insgesamt |
| 248,2 | g | Butylglykol, homogenisiert bei 150 °C bis zum Verbrauch der freien NCO-Gruppen. Das hellbraune Produkt zeigt im IR-Spektrum u.a. die für Hydantoine typischen Absorbtionsbanden und besitzt eine Viskosität von 210 cP bei 150 °C. |

b)

| | | |
|---|---|---|
| 100 | g | des Harzes 8a) werden mit |
| 500 | g | eines Polyesters aus |
| | | 1106 g Dimethylterephthalat, |
| | | 493 g Tris-(2-hydroxyäthyl)-isocyanurat, |
| | | 87 g Trimethylolpropan, |
| | | 620 g Äthylenglykol, |
| | | 100 g Xylol, |
| | | 200 g Solvesso und |
| | | 1,5 g Bleiacetat mit einem OH-Gehalt von 4,5 Gew.-%, bei 150–175 °C verschmolzen. |

Das resultierende Harz besitzt eine Viskosität von ca. 2700 cP bei 170 °C.

Es kann entweder direkt aus der Schmelze bei einer Badtemperatur von ca. 160–170 °C oder beispielsweise aus einer ca. 35 gewichtsprozentigen Lösung in Benzylalkohol mit einer Lösungsviskosität von ca. 2450 mPa·$s_{20°C}$ zur Beschichtung hitzeresistenter Substrate verwendet werden.

So besitzen die aus der obigen Schmelze oder aus der obigen Lösung in einem 4 m-Ofen bei 400 °C auf einem Cu-Draht von 0,7 mm eingebrannten Lackfilme eine Erweichungstemperatur von >330 °C, einen Hitzeschock von mindestens 240 °C, eine Wärmealterung von mindestens 7 Tagen 200 °C bei hoher Elastizität, guter Schabefestigkeit und Lösungsmittelbeständigkeit.

**Patentansprüche**

1. Polyiso(thio)cyanate, die mindestens einen mit Estergruppen modifizierten Hydantoinring enthalten, der allgemeinen Formel (I)

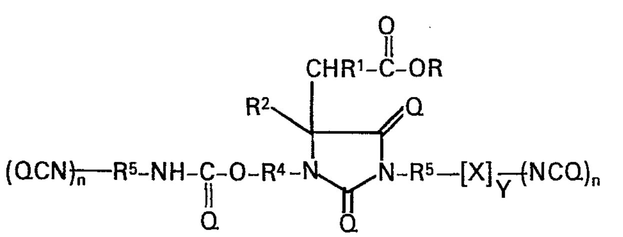 (I)

in der X für die wiederkehrenden Einheiten (II) und/oder (III)

(II) 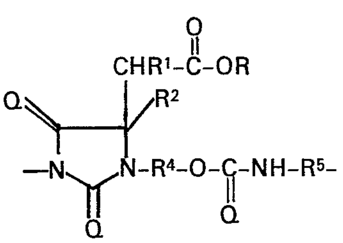 (III) 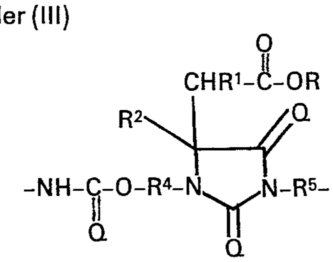

steht,
R für einen aliphatischen Rest mit $C_1$–$C_{20}$, der über Äther-, Ester-, Urethan-, Harnstoff-, Amid-, Imid-, Amidimid-, Esterimid-, Esteramidimid-, Epoxidgruppen in Polymere eingebaut sein kann oder mit $R^1$ bzw. $R^2$ einen Ring mit bis zu 8 Ringgliedern bildet, steht,
$R_1$ und $R_2$ gleich oder verschieden, Wasserstoff, Halogen, einen Alkylrest mit $C_1$–$C_6$ oder Cycloalkylrest mit $C_5$–$C_8$, Alkylarylrest mit $C_7$–$C_{16}$, einen

Arylrest mit $C_6$–$C_{20}$ bedeuten oder zusammen einen Ring mit bis zu 8 Ringgliedern bilden,

$R_4$ für einen aliphatischen Rest mit $C_2$–$C_{10}$, einen cycloaliphatischen Rest mit $C_4$–$C_{16}$, einen araliphatischen Rest mit $C_7$–$C_{16}$, einen aromatischen Rest mit $C_6$–$C_{20}$, oder einen ein oder mehrere Heteroatome wie N, O oder S enthaltenden Aryl- oder Cycloalkylrest mit $C_5$–$C_{12}$, die alle mit Halogen- und/oder Alkylgruppen mit $C_1$–$C_6$ substituiert sein können, steht,

$R_5$ für einen aliphatischen Rest mit 2–20 C-Atomen, einen aromatischen Rest mit 5–12 C-Atomen, einen cycloaliphatischen Rest mit 5–12 C-Atomen, einen aliphatisch-aromatischen Rest mit 6–20 C-Atomen oder einen ein oder mehrere Heteroatome wie N, O oder S enthaltenden aromatischen oder cycloaliphatischen Rest mit 5–12 C-Atomen steht, die alle durch Halogen, Alkyl- mit $C_1$–$C_6$ und/oder Arylgruppen mit $C_6$–$C_{16}$ substituiert sein können,

Y 0 oder eine ganze Zahl von 1–20,

Q Sauerstoff oder Schwefel und

n eine ganze Zahl von 1–3 bedeuten.

2. Verfahren zur Herstellung von Polyiso(thio)cyanaten gemäss Anspruch 1, durch Umsetzung von gegebenenfalls substituierten, gegebenenfalls in Polymeren eingebauten Äthylen-1,2-dicarbonsäureestern mit primären Aminoalkoholen zu den entsprechenden Asparaginsäureester und gegebenenfalls gleichzeitige und/oder anschliessende Reaktion mit Polyisocyanaten, dadurch gekennzeichnet, dass pro Mol Asparaginsäureester mindestens 2,1 Äquivalente Isocyanat eingesetzt werden.

3. Verfahren zur Herstellung von Polyiso(thio)cyanaten gemäss Anspruch 3, dadurch gekennzeichnet, dass pro Mol Aminoalkohol mindestens 2,1 Äquivalente Polyiso(thio)cyanat eingesetzt werden.

4. Verfahren gemäss Ansprüchen 2 und 3, dadurch gekennzeichnet, dass als Äthylen-1,2-dicarbonester Ester der allgemeinen Formel V verwendet werden,

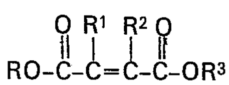 (V)

in der

R, $R^1$ und $R^2$ die in dem Anspruch 1 angegebene Bedeutung haben, und

$R^3$ die Bedeutung von R hat, aber nicht identisch mit R sein muss.

5. Verfahren gemäss Anspruch 4, dadurch gekennzeichnet, dass als Äthylen-1,2-dicarbonsäureester Ester der Malein- oder Fumarsäure mit Methanol, Äthanol, Butanolen, Benzylalkoholen, Phenolen oder Glykoläther verwendet werden.

6. Verfahren gemäss Ansprüchen 2–5, dadurch gekennzeichnet, dass als primäre Aminohydroxyisoverbindungen Verbindungen der allgemeinen Formel IV verwendet werden,

$$HO\text{-}R^4\text{-}NH_2 \qquad (IV)$$

in der

$R^4$ die in dem Anspruch 1 angegebene Bedeutung hat.

7. Verfahren gemäss Anspruch 6, dadurch gekennzeichnet, dass als Aminohydroxyverbindung Äthanolamin, Propanolamin, Butanolamin oder Aminophenol verwendet werden.

8. Verfahren gemäss Ansprüche 2–7, dadurch gekennzeichnet, dass als Polyiso(thio)cyanate solche der allgemeinen Formel VII verwendet werden,

$$R^5(NCQ)_{n+1} \qquad (VII)$$

in der

$R^5$ und Q die in dem Anspruch 1 angegebene Bedeutung haben.

9. Verwendung der Polyiso(thio)cyanate gemäss Anspruch 1 zur Herstellung von hitzebeständigen Beschichtungsmaterialien, Folien, Kleber, Pulver und Formkörper.

## Revendications

1. Des polyiso(thio)cyanates qui renferment au moins un noyau d'hydantoïne modifié par des groupes ester, de formule générale I:

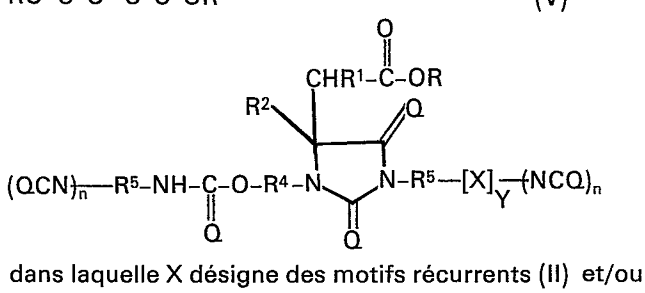 (I)

dans laquelle X désigne des motifs récurrents (II) et/ou (III):

(II) 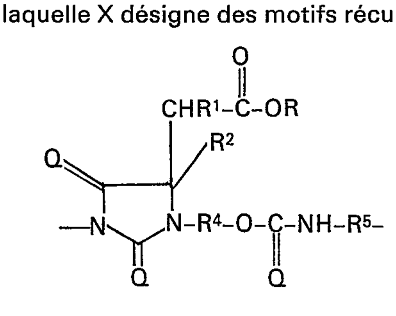

(III) 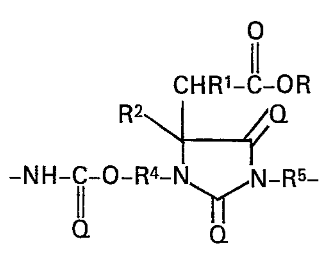

R est un reste aliphatique ayant 1 à 20 atomes de carbone, qui peut être incorporé à des polymères par l'intermédiaire de groupes éther, ester, uréthanne, urée, amide, imide, amido-imide, esterimide, esteramido-imide, époxyde, ou forme avec $R^1$ ou $R^2$ un noyau ayant jusqu'à 8 chaînons,
$R^1$ et $R^2$ égaux ou différents, représentent de l'hydrogène, un halogène, un reste alkyle ayant 1 à 6 atomes de carbone ou un reste cycloalkyle ayant 5 à 8 atomes de carbone, un reste alkylaryle ayant 7 à 16 atomes de carbone, un reste aryle ayant 6 à 20 atomes de carbone, ou forment ensemble un noyau ayant jusqu'à 8 chaînons,
$R^4$ représente un reste aliphatique ayant 2 à 10 atomes de carbone, un reste cycloaliphatique ayant 4 à 16 atomes de carbone, un reste araliphatique ayant 7 à 16 atomes de carbone, un reste aromatique ayant 6 à 20 atomes de carbone ou un reste aryle ou cycloalkyle renfermant un ou plusieurs hétéro-atomes tels que N, O ou S ayant 5 à 12 atomes de carbone, qui peuvent tous être substitués avec des halogènes et/ou des groupes alkyle ayant 1 à 6 atomes de carbone,
$R^5$ représente un reste aliphatique ayant 2 à 20 atomes de carbone, un reste aromatique ayant 5 à 12 atomes de carbone, un reste cycloaliphatique ayant 5 à 12 atomes de carbone, un reste aliphatique-aromatique ayant 6 à 20 atomes de carbone ou un reste aromatique ou cycloaliphatique renfermant un ou plusieurs hétéro-atomes tels que N, O ou S et ayant 5 à 12 atomes de carbone, qui peuvent tous être substitués par un halogène, des groupes alkyle en $C_1$ à $C_6$ et/ou des groupes aryle en $C_6$ à $C_{16}$,
Y est égal à 0 ou à un nombre entier de 1 à 20,
Q est de l'oxygène ou du soufre, et
n est un nombre entier de 1 à 3.

2. Procédé de production de polyiso(thio)cyanates suivant la revendication 1, par réaction d'esters d'acide éthylène-1,2-dicarboxylique éventuellement substitués, le cas échéant incorporés à des polymères, avec des amino-alcools primaires pour former les esters d'acide asparagique correspondants et, le cas échéant, par réaction simultanée et/ou subséquente avec des polyiso(thio)cyanates, caractérisé en ce qu'on utilise au moins 2,1 équivalents d'isothiocyanate par mole d'ester d'acide asparagique.

3. Procédé de production de polyiso(thio)cyanates suivant la revendication 2, caractérisé en ce qu'on utilise, par mole d'amino-alcool, au moins 2,1 équivalents de polyiso(thio)cyanate.

4. Procédé suivant les revendications 2 et 3, caractérisé en ce qu'on utilise, comme ester d'acide éthylène-1,2-dicarboxylique, un ester de formule générale V:

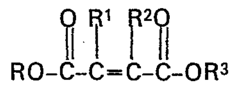
(V)

dans laquelle:
R, $R^1$ et $R^2$ ont la définition donnée dans les revendications 1 et 2, et
$R^3$ a la définition de R, mais ne doit pas être identique à R.

5. Procédé suivant la revendication 4, caractérisé en ce qu'on utilise, comme ester d'acide éthylène-1,2-dicarboxylique, un ester d'acide maléique ou d'acide fumarique avec le méthanol, l'éthanol, des butanols, des alcools benzyliques, des phénols ou un éther de glycol.

6. Procédé suivant les revendications 2 à 5, caractérisé en ce qu'on utilise, comme composés amino hydroxylés primaires, des composés de formule générale IV:

$$HO-R^4-NH_2 \quad (IV)$$

dans laquelle:
$R^4$ a la définition donnée dans la revendication 1.

7. Procédé suivant la revendication 6, caractérisé en ce qu'on utilise, comme composé amino hydroxylé, l'éthanolamine, la propanolamine, la butanolamine ou l'aminophénol.

8. Procédé suivant les revendications 2 à 7, caractérisé en ce qu'on utilise comme polyiso(thio)cyanates des composés de formule générale VII:

$$R^5(NCQ)_{n+1} \quad (VII)$$

dans laquelle:
$R^5$ et Q ont la définition donnée dans la revendication 1.

9. Utilisation des polyiso(thio)cyanates suivant la revendication 1 pour la production de matières d'enduction, de feuilles, d'adhésifs, de poudres et de corps moulés stables à la chaleur.

**Patent Claims**

1. Polyiso(thio)cyanates which contain at least one hydantoin ring modified by ester groups, of the general formula I

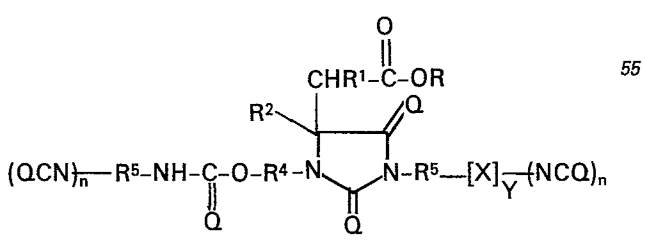
(I)

in which X represents the recurrent units (II) and/or (III)

(II) 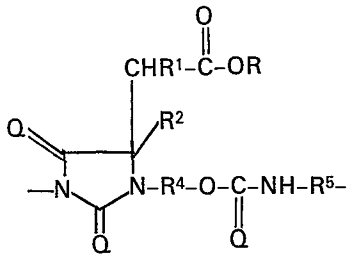

(III) 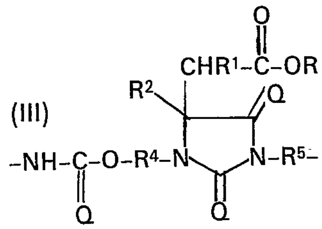

R represents a $C_1$–$C_{20}$ aliphatic radical which may be incorporated into polymers via ether, ester, urethane, urea, amide, imide, amido imide, ester imide, ester amido imide, or epoxide groups or together with $R^1$ or $R^2$ forms a ring with up to 8 ring members,
$R_1$ and $R_2$, which may be the same or different, represent hydrogen, halogen, a $C_1$–$C_6$ alkyl group, or $C_5$–$C_8$ cycloalkyl group, $C_7$–$C_{16}$ alkyl aryl group, or a $C_6$–$C_{20}$ aryl group, or together form a ring with up to 8 ring members,
$R_4$ represents a $C_2$–$C_{10}$ aliphatic group, a $C_4$–$C_{16}$ cycloaliphatic group, a $C_7$–$C_{16}$ araliphatic group, a $C_6$–$C_{20}$aromatic group or a $C_5$–$C_{12}$ aryl or cycloalkyl group containing one or more heteroatoms such as N, O or S, all of which groups may be substituted by halogen and/or alkyl groups with $C_1$–$C_6$,
$R_5$ represents an aliphatic group with 2–20 C atoms, an aromatic group with 5–12 C atoms, a cycloaliphatic group with 5–12 C atoms, an aliphatic-aromatic group with 6–20 C atoms or an aromatic or cycloaliphatic group with 5–12 C atoms containing one or more heteroatoms such as N, O or S, all of which may be substituted by halogen, $C_1$–$C_6$ alkyl and/or $C_6$–$C_{16}$aryl groups,
Y denotes 0 or an integer from 1-20,
Q denotes oxygen or sulphur and
n denotes an integer from 1-3.

2. Process for the preparation of polyiso(thio)cyanates according to claim 1, by reacting optionally substituted, ethylene-1,2-dicarboxylic acid esters, which are optionally incorporated in polymers, with primary amino alcohols to form the corresponding aspartic acid esters and optionally simultaneously and/or subsequently reacting the aspartic acid esters with polyiso(thio)cyanates, characterised in that at least 2.1 equivalents of isothiocyanate are used per mole of aspartic acid ester.

3. Process for the preparation of polyiso(thio)cyanates according to claim 2, characterised in that at least 2.1 equivalents of polyiso(thio)cyanate are used per mole of amino alcohol.

4. Process according to claims 2 and 3, characterised in that esters of the general formula V are used as the ethylene-1,2-dicarboxylic acid esters

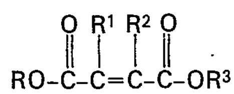 (V)

in which
R, $R^1$ and $R^2$ have the meaning given in claims 1 and 2, and
$R^3$ has the meaning of R but does not have to be identical to R.

5. Process according to claim 4, characterised in that esters of maleic or fumaric acid with methanol, ethanol, butanols, benzyl alcohols, phenols or glycol ethers are used as the ethylene-1,2-dicarboxylic acid esters.

6. Process according to claims 2 to 5, characterised in that compounds of the general formula IV

$$HO\text{-}R^4\text{-}NH_2 \qquad (IV)$$

in which
$R^4$ has the meaning given in claim 1, are used as the primary aminohydroxy compounds.

7. Process according to claim 6, characterised in that ethanolamine, propanolamine, butanolamine or aminophenol are used as the aminohydroxy compound.

8. Process according to claims 2 to 7, characterised in that the polyiso(thio)cyanates used are those of the general formula VII

$$R^5(NCQ)_{n+1} \qquad (VII),$$

in which
$R^5$ and Q have the meaning as defined in claim 1.

9. Use of the polyiso(thio)cyanates according to claim 1 for the preparation of heat-resistant coating materials, foils, adhesives, powders and shaped products.